# FASCICULE DE BREVET EUROPEEN

(11) **EP 2 263 614 B1**
(45) Date de publication et mention de la délivrance du brevet: **19.12.2012**
(21) Numéro de dépôt: 10166447.2
(22) Date de dépôt: 18.06.2010
(51) Int. Cl.: A61F 2/42, A61B 17/56

(54) **Implant d'articulation trapézo-métacarpienne**
Trapezo-Metakarpales Gelenkimplantat
Trapezo-metacarpal joint implant

(30) Priorité: 19.06.2009 FR 0954190
(43) Date de publication de la demande: 22.12.2010
(73) Titulaire: Tornier, 38330 Montbonnot-Saint-Martin (FR)
(72) Inventeur: Hassler, Michel, 38330, SAINT ISMIER (FR); Bellemere, Philippe, 44000, NANTES (FR)
(74) Mandataire: Grand, Guillaume

(56) Documents cités:
- WO-A-97/42895
- WO-A-2004/093767
- US-A1- 2006 241 758
- US-B1- 6 436 146

## Description

La présente invention concerne un implant d'articulation trapézo-métacarpienne.

Les prothèses et les implants trapézo-métacarpiens sont destinés à rétablir la force et la mobilité de l'articulation anatomique du pouce, entre son métacarpien et le trapèze lorsque cette articulation est endommagée par un processus pathologique dégénératif ou inflammatoire. L'invention s'intéresse ainsi plus particulièrement aux implants trapézo-métacarpiens destinés à traiter une arthrose peu destructrice et relativement centrée.

Les prothèses trapézo-métacarpiennes connues sont des prothèses à tige, c'est-à-dire dont le composant prothétique métacarpien est, d'un côté, ancré dans le métacarpien par une tige allongée, tandis que, de l'autre côté, ce composant prothétique est monté rotulant soit dans un composant prothétique rapporté fixement au trapèze, soit dans une cavité creusée directement dans le trapèze. L'implantation de ces prothèses totales ou partielles nécessitent donc d'entamer considérablement la matière osseuse de l'articulation trapézo-métacarpienne et rétablit une mobilité de type rotule qui ne correspond pas, d'un point de vue cinématique, à la mobilité articulaire anatomique. De plus, en cas de mauvaise implantation, les risques d'usure, de luxation, voire de fracture de la prothèse sont réels.

On connaît par ailleurs, par exemple de US-B-6 436 146, des implants trapézo-métacarpiens sans tige, constitués d'un corps monobloc sphérique ou ellipsoïdal, logé dans une cavité concave creusée de façon complémentaire dans les os du trapèze et du métacarpien. Pour assurer la stabilité de ce corps d'implant, la cavité doit être creusée de manière assez profonde dans le trapèze et le métacarpien, à travers leurs couches osseuses corticales, jusqu'à atteindre la matière spongieuse. Il en résulte que, à la longue, en raison des contraintes de compression appliquées au corps d'implant par l'environnement ligamentaire de l'articulation, ce corps s'enfonce progressivement dans les os, ce qui réduit l'écartement entre ces os et donc leur mobilité relative, jusqu'à les fusionner le cas échéant. De plus, ce corps prothétique fournit une mobilité de type sphérique ou ellipsoïdal entre le trapèze et le métacarpien, ce qui n'est pas satisfaisant d'un point de vue cinématique.

Le but de la présente invention est de proposer un implant trapézo-métacarpien, dont l'implantation est pérenne et qui fournit une mobilité plus proche de la mobilité anatomique de l'articulation trapézo-métacarpienne.

A cet effet, l'invention a pour objet un implant d'articulation trapézo-métacarpienne, tel que défini à la revendication 1.

Est également présentée ici une méthode chirurgicale de pose d'un implant d'articulation trapézo-métacarpienne tel que défini ci-dessus, dans laquelle :
- on accède à la zone articulaire entre le métacarpien et le trapèze d'un patient,
- on prépare la surface osseuse corticale du trapèze de manière sensiblement complémentaire à la surface articulaire trapézienne définie par le corps de l'implant et, si besoin, on prépare la surface osseuse corticale du métacarpien de manière sensiblement complémentaire à la surface articulaire métacarpienne définie par le corps de l'implant, et
- on interpose le corps de l'implant entre le trapèze et le métacarpien de manière que l'axe associé à la surface articulaire trapézienne s'étend dans l'un des plans antéro-postérieur et frontal du patient, tandis que l'axe associé à la surface articulaire métacarpienne s'étend dans l'autre de ces plans.

L'idée à la base de l'invention est d'interposer entre le trapèze et le métacarpien un corps non pas volumineux et sphérique ou ellipsoïdal, mais un corps relativement fin et avec des surfaces articulaires opposées, qui sont concaves au moins dans une direction et contre lesquelles vont s'appuyer pour rouler et glisser les surfaces osseuses corticales du trapèze et du métacarpien. Pour reproduire aussi fidèlement que possible le comportement anatomique de l'articulation trapézo-métacarpienne, de type selle de cheval, les deux surfaces articulaires sont réalisées par des segments axiaux de cylindre, de cône et/ou de tore, dont les axes centraux ou, plus généralement, pour tenir compte du fait que l'axe central d'un segment axial de tore est courbe, les projections de ces axes centraux sur le plan médian de l'implant sont sensiblement perpendiculaires l'une à l'autre : le corps de l'implant conforme à l'invention dissocie la selle de cheval anatomique en ces deux surfaces articulaires. D'un point de vue cinématique, ces deux surfaces articulaires orthogonales autorisent une mobilité pivotante autour des deux axes centraux précités, à la façon d'un joint de cardan auquel l'articulation trapézo-métacarpienne est assimilée d'un point de vue de mécanique théorique. De plus, la courbure de ces deux surfaces articulaires stabilise et aligne le corps de l'implant entre le trapèze et le métacarpien, ce qui évite de prévoir des moyens de fixation osseuse. En outre, l'invention présente l'avantage de maximiser l'étendue du contact entre l'implant et les os du trapèze et du métacarpien, au niveau de ces deux surfaces articulaires. Il en résulte que l'implant ne s'enfonce pas dans les os, préservant ainsi sa mobilité.

En pratique, le corps de l'implant selon l'invention présente une épaisseur la plus petite possible, étant entendu qu'une gamme de plusieurs épaisseurs peut être proposée selon le degré d'usure de l'articulation à traiter. Cette faible épaisseur vise à limiter au maximum les découpes osseuses nécessaires pour rendre complémentaire les surfaces osseuses corticales en regard du trapèze et du métacarpien avec les surfaces articulaires du corps de l'implant, par ajustement de la conformation de ces surfaces osseuses corticales, étant remarqué que, dans leur état anatomique non altéré, les surfaces osseuses corticales du trapèze et du métacarpien sont proches de conformations optimales pour coopérer avec le corps de l'implant. D'ailleurs, pour certaines articulations trapézo-métacarpiennes peu altérées, la recoupe sur le métacarpien peut être minime, voire nulle. Dans tous les cas, la taille de l'implant doit permettre de stabiliser immédiatement son corps par la seule contrainte de l'enveloppe capsulaire et ligamentaire autour de l'articulation entre le trapèze et le métacarpien, sans pour autant que ce corps subisse une hyper-contrainte puisqu'il doit conserver une certaine liberté afin que sa position s'adapte en fonction des contraintes de l'enveloppe articulaire, liées aux différents mouvements possibles de cette articulation.

D'autres caractéristiques avantageuses de l'implant conforme à l'invention, prises isolément ou selon toutes les combinaisons techniquement possibles, sont spécifiées aux revendications dépendantes 2 à 10.

L'invention sera mieux comprise à la lecture de la description qui va suivre, donnée uniquement à titre d'exemple et faite en se référant aux dessins sur lesquels :
- la figure 1 est une vue en élévation représentant certains des constituants osseux d'une main humaine gauche, dans laquelle est implanté un implant conforme à l'invention ;
- la figure 2 est une vue en perspective de l'implant de la figure 1 ;
- la figure 3 est une vue en élévation selon la flèche III de la figure 2 ;
- les figures 4 et 5 sont des coupes selon respectivement les lignes IV-IV et V-V de la figure 3, montrant également en partie les os de la main après mise en place de l'implant ;
- la figure 6 est un schéma illustrant la géométrie de construction des surfaces articulaires de l'implant des figures 1 à 5 ; et
- les figures 7 à 9 sont des vues analogues à la figure 6, illustrant respectivement des variantes pour la géométrie de construction des surfaces articulaires de l'implant conforme à l'invention.

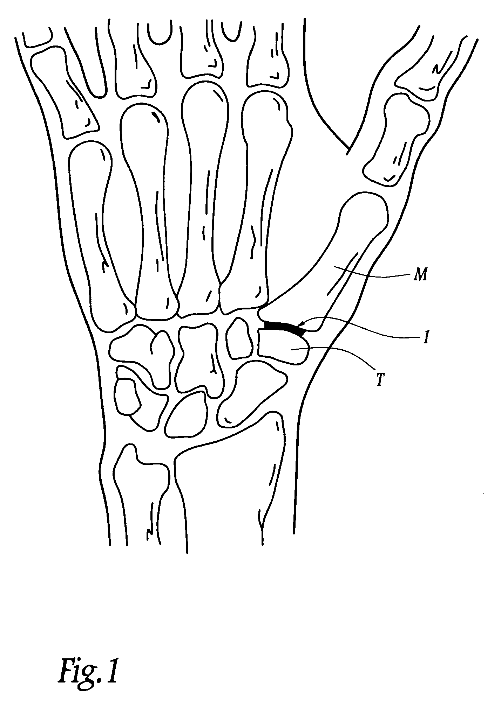

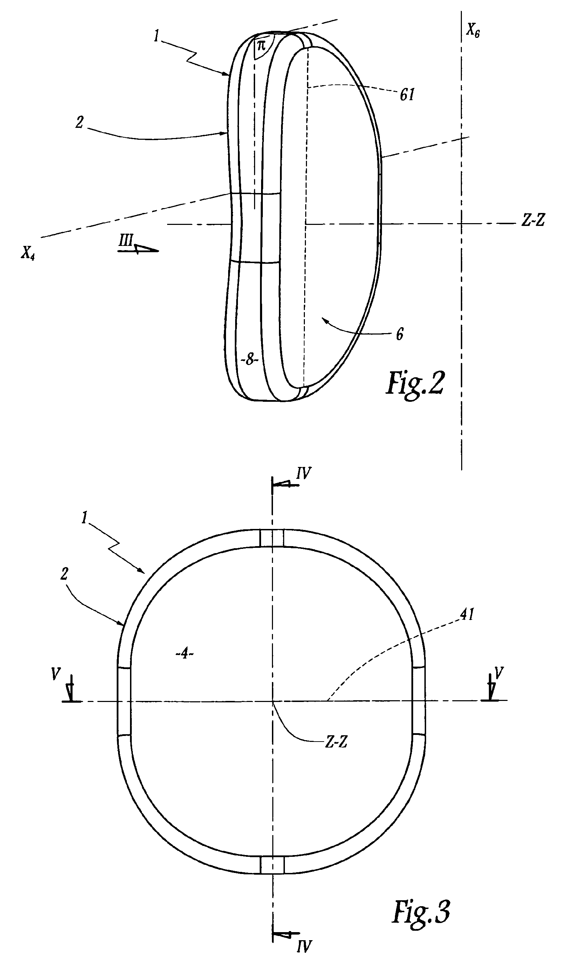

Sur les figures 1 à 5 est représenté un implant trapézo-métacarpien 1 adapté pour être implanté de manière interposée entre le trapèze T et le premier métacarpien M d'une main humaine, telle que celle montrée sur la figure 1 à titre d'exemple.

L'implant 1 comporte un corps 2 globalement discoïdal, centré sur un axe géométrique référencé Z-Z. Le corps 2 délimite ainsi, d'une part, deux surfaces principales 4 et 6 opposées selon l'axe Z-Z, qui sont séparées l'une de l'autre par l'épaisseur, selon cet axe Z-Z, du corps 2, et, d'autre part, une surface périphérique 8, qui relie l'une à l'autre les surfaces 4 et 6 et dont les génératrices, dans l'exemple de réalisation considéré ici, sont parallèles à l'axe Z-Z. En coupe transversale à l'axe Z-Z, le corps 2 présente un contour extérieur rectangulaire aux coins émoussés. En variantes non représentées, d'autres formes sont envisageables pour ce contour, telles qu'un carré, un rond, une ellipse, etc.

Dans la forme de réalisation envisagée sur les figures, l'implant 1 n'est constitué que du corps 2, dans le sens où l'implant ne comporte aucun autre composant, notamment aucun composant de fixation osseuse qui permettrait d'ancrer fixement le corps 2 dans le métacarpien M ou dans le trapèze T. Dans ce cas, le corps 2 constitue un implant exclusivement d'interposition de manière que ce corps présente, après implantation entre le métacarpien et le trapèze, une certaine liberté de mouvement, sa position s'adaptant aux contraintes qui lui sont appliquées, en fonction des mouvements de l'articulation trapézo-métacarpienne.

Dans cette optique, le corps 2 est avantageusement réalisé d'un seul tenant en un bloc de graphite recouvert de carbone pyrolytique, ce qui confère à ce corps une bonne biocompatibilité, une haute résistance mécanique et une bonne tenue à l'usure vis-à-vis des os métacarpien et trapèze. En pratique, d'autres matériaux peuvent être utilisés, notamment un alliage métallique à base de chrome et de cobalt, une matière plastique, comme le polyéthylène et le PEEK, du silicone, de la céramique, etc.

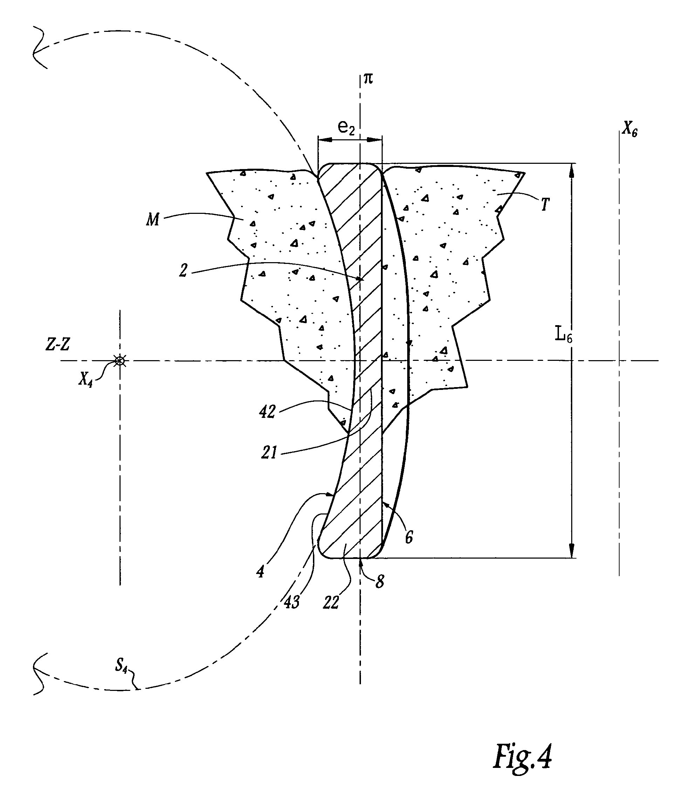

La surface 4 est concave. Elle correspond géométriquement à une portion de cylindre, qui est centrée sur un axe X₄ et dont la section transversale S₄, qui est constante suivant l'axe X₄ et qui correspond donc à la directrice de la surface 4, est sensiblement elliptique, comme bien visible sur la figure 4. L'axe X₄ s'étend de manière perpendiculaire à l'axe Z-Z, ces axes étant agencés de manière séquente dans l'exemple de réalisation considéré ici.

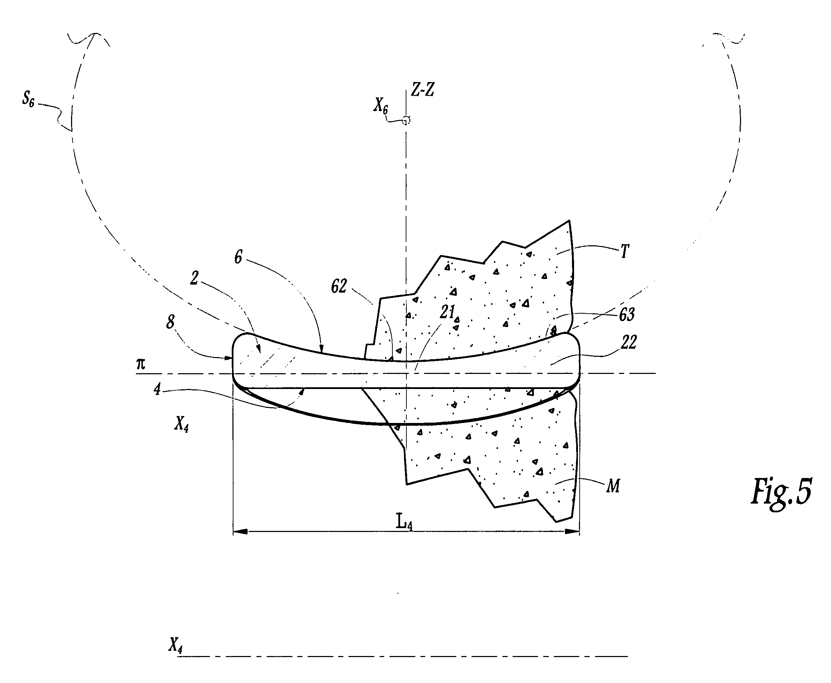

De même, la surface 6 est concave et correspond à une portion de cylindre, dont la section transversale S₆ est sensiblement elliptique et qui est centrée sur un axe X₆ perpendiculaire, ici de manière séquente, avec l'axe Z-Z, comme bien visible sur la figure 5.

Ainsi, les axes X₄ et X₆ s'étendent de manière perpendiculaire l'un à l'autre, de manière non séquente. En particulier, les lignes de fond 41 et 61, qui appartiennent respectivement aux surfaces 4 et 6 et qui s'étendent respectivement parallèlement aux axes X₄ et X₆, s'étendent de manière perpendiculaire l'une à l'autre, en étant séparées, selon l'axe Z-Z, par la partie centrale 21 du corps 2. Autrement dit, dans un plan médian π du corps 2, qui est perpendiculaire à l'axe Z-Z et de part et d'autre duquel sont situées les surfaces 4 et 6, les projections respectives de ces axes X₄ et X₆ sont perpendiculaires et sécantes.

De par les caractéristiques géométriques des surfaces 4 et 6, on comprend que, en coupe transversale passant par l'axe Z-Z, le corps 2 présente une épaisseur variable, en étant minimale dans la partie centrale 21 du corps tandis qu'elle est progressivement croissante en s'éloignant de cette partie centrale 21, jusque dans la partie périphérique 22 du corps 2, comme bien visible sur les figures 4 et 5.

D'autres caractéristiques de l'implant 1 apparaitront ci-après, dans le cadre de la description d'un exemple de mise en place de cet implant entre le métacarpien M et le trapèze T montrés à la figure 1.

Dans un premier temps opératoire, un chirurgien accède à la zone articulaire entre le métacarpien M et le trapèze T, via une voie d'abord postérieure ou antéro-latérale. Pour accéder à l'espace inter-osseux entre le métacarpien et le trapèze, la capsule articulaire et les ligaments entourant cette zone articulaire sont soit respectés, en étant écartés, soit partiellement incisés, étant entendu que cette capsule et ses ligaments seront alors reconstruits en fin d'intervention.

Dans un deuxième temps opératoire, le chirurgien écarte l'un de l'autre le métacarpien M et le trapèze T afin d'élargir l'espace inter-osseux, notamment en exerçant un effort de traction suivant la direction longitudinale du métacarpien. Le chirurgien peut alors préparer les surfaces osseuses en regard délimitées par le métacarpien M et le trapèze T, de manière à les conformer de façon complémentaire aux surfaces 4 et 6 du corps 2 de l'implant 1.

En pratique, on notera que les extrémités en regard du métacarpien M et du trapèze T présentent des géométries de surface respectives proches de surfaces complémentaires aux surfaces 4 et 6. En effet, ces extrémités osseuses présentent, dans leur état anatomique, des formes en selle de cheval, qui s'emboîtent orthogonalement l'une dans l'autre. Ainsi, les découpes osseuses réalisées sur les extrémités en regard du métacarpien et du trapèze sont minimes pour adapter la surface de ces os aux surfaces 4 et 6 du corps 2. D'ailleurs, en pratique, ces découpes sont minimes voire inutiles sur le métacarpien, tandis que, en général, des découpes plus importantes doivent être réalisées sur le trapèze. Ainsi, la préparation des extrémités du métacarpien M et du trapèze T n'entame pas profondément ces os, en restant au niveau de leur couche corticale osseuse.

Dans un troisième temps opératoire, tout en maintenant la traction sur l'articulation trapézo-métacarpienne selon la direction longitudinale du métacarpien M, le chirurgien positionne l'implant 1 dans l'espace inter-osseux séparant le métacarpien et le trapèze T, en interposant le corps 2 entre ces deux os de manière que la surface 4 est tournée vers le métacarpien, avec l'axe X₄ s'étendant dans un plan frontal, tandis que la surface 6 est tournée vers le trapèze, avec l'axe X₆ qui s'étend dans un plan antéro-postérieur, étant entendu que les termes « frontal », « antéro-postérieur » et similaires s'entendent ici dans leur sens anatomique, par rapport au patient dont la main est opérée. En variante non représentée, l'implant 1 est mis en place, entre le métacarpien M et la trapèze T, dans une configuration basculée de 90° autour de l'axe Z-Z : une préparation préalable des extrémités du métacarpien et du trapèze peut être nécessaire, mais cette variante illustre la capacité du corps 2 à être implanté de façon que ses surfaces articulaires 4 et 6 s'adaptent au mieux aux extrémités du métacarpien et du trapèze du patient opéré, selon l'état initial de ces extrémités osseuses et/ou pour minimiser la profondeur d'entame de ces extrémités lors de leur préparation.

Une fois que l'implant 1 est ainsi positionné entre le métacarpien M et le trapèze T, la traction axiale précitée est relâchée, de sorte que la capsule articulaire et les ligaments entourant l'articulation trapézo-métacarpienne rapprochent l'un de l'autre le métacarpien et le trapèze. Le corps 2 de l'implant 1 se trouve alors retenu de manière mobile entre le métacarpien et le trapèze, sous la contrainte de la capsule et des ligaments, autrement dit sous une contrainte dont la résultante est sensiblement alignée avec l'axe Z-Z. Le chirurgien peut ensuite refermer les tissus mous autour de l'articulation, le cas échéant par reconstruction, voire moyennant une ligamentoplastie.

L'articulation trapézo-métacarpienne ainsi munie de l'implant 1 retrouve un comportement cinématique proche, voire quasi-identique aux comportements anatomiques d'origine de cette articulation. En effet, le métacarpien M s'articule alors contre la surface 4, essentiellement par basculement autour de l'axe X₄, tandis que, dans le même temps, le trapèze T s'articule contre la surface 6, essentiellement par basculement autour de l'axe X₆. Grâce à l'implant 1, le métacarpien M et le trapèze T sont articulés l'un par rapport à l'autre à la façon d'un joint de cardan, autour des deux axes perpendiculaires X₄ et X₆. Cette cinématique de joint de cardan reproduit efficacement l'articulation anatomique sellaire entre le métacarpien et le trapèze. De plus, on notera que les surfaces 4 et 6 s'appuient alors contre les couches corticales osseuses respectives du métacarpien M et du trapèze T, ce qui évite que le corps 2 ne s'enfonce dans l'un et/ou l'autre de ces os. La mobilité fournie par l'implant 1 est ainsi pérenne.

Avantageusement, pour favoriser les mouvements de roulement et de glissement du métacarpien M et du trapèze T contre respectivement les parties centrales 42 respectives des surfaces 4 et 6, la section elliptique S₄, S₆ de ces surfaces présente, dans ces régions centrales 42, 62, une courbure plus petite que le reste de la section. Autrement dit, les régions centrales 42 et 62 des surfaces 4 et 6 sont alors un peu plus aplaties, c'est-à-dire un peu moins courbées que le reste de ces surfaces.

Par ailleurs, en service, le corps 2 est stabilisé entre le métacarpien M et le trapèze T du fait de la courbure elliptique des sections respectives S₄, S₆ des surfaces 4 et 6. Avantageusement, pour renforcer cette stabilité, la section des régions périphériques respectives 43 et 63 des surfaces 4 et 6 présente une courbure plus grande que le reste de cette section : en exagérant ainsi la courbure des surfaces 4 et 6 au niveau de leur bordure d'extrémité périphérique, la coopération surfacique du corps 2 et des os du métacarpien M et du trapèze T auto-stabilise l'implant 1. De surcroît, les régions périphériques 43 et 63 des surfaces 4 et 6 peuvent ainsi compenser l'usure périphérique, liée à l'arthrose, des extrémités osseuses en regard du métacarpien et du trapèze.

En pratique, on comprend que, dans la mesure où le corps 2 est un implant d'interposition, son épaisseur suivant l'axe Z-Z est limitée, dans le sens où la présence de ce corps ne doit pas entraîner une hypercontrainte de l'articulation trapézo-métacarpienne. Ainsi, à titre d'exemple numérique, l'épaisseur maximale e₂ du corps 2, c'est-à-dire son épaisseur entre les régions périphériques 43 et 63 des surfaces 4 et 6, est prévue inférieure à 5 mm, en particulier jusqu'à être égale à 1 mm environ.

De même, pour s'adapter au mieux à l'espace inter-osseux entre le métacarpien M et le trapèze T, la surface 6 présente avantageusement une dimension L₆, suivant son axe X₆, supérieure à la dimension L₄, suivant l'axe X₄, de la surface 4.

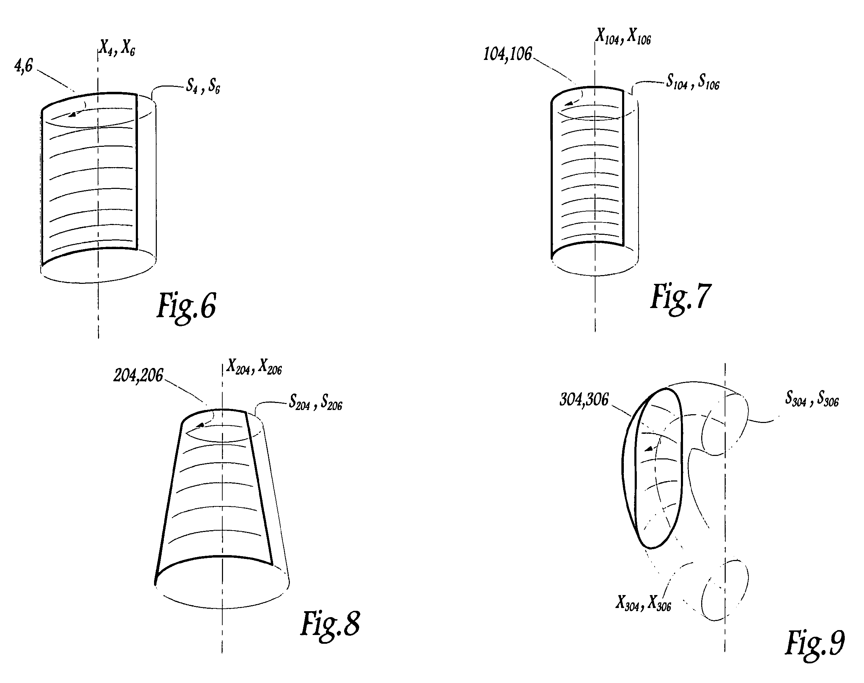

Par ailleurs, la géométrie des surfaces articulaires métacarpien et trapèze peut être construite de manière différente de celle des surfaces 4 et 6 décrites jusqu'ici. Pour bien comprendre cet aspect de l'invention, on a représenté, sur la figure 6, un schéma permettant de visualiser la géométrie de construction des surfaces 4 et 6 : comme bien visible sur cette figure 6, chaque surface 4, 6 correspond à une portion, autrement dit à un segment axial, d'un cylindre d'axe central X₄, X₆, à base elliptique, c'est-à-dire avec sa section S₄, S₆ en forme d'ellipse centrée sur l'axe X₄, X₆. Ainsi, on retrouve bien ici la définition géométrique donnée plus haut pour les surfaces 4 et 6.

Sur les figures 7, 8 et 9 sont représentés des schémas similaires à celui de la figure 6, de manière à illustrer des variantes pour les surfaces articulaires 4 et 6 de l'implant 1. Ainsi, sur la figure 7, on propose des surfaces articulaires métacarpienne 104 et trapézienne 106, qui correspondent chacune à un segment axial d'un cylindre, centré sur un axe X₁₀₄, X₁₀₆ et dont la section transversale S₁₀₄, S₁₀₆ est circulaire centrée sur l'axe précité. Autrement dit, les surfaces 4 et 6 et les surfaces 104 et 106 se distinguent uniquement par la forme géométrique de leur section transversale, à savoir elliptique pour les surfaces 4 et 6 et circulaire pour les surfaces 104 et 106.

De même, sur la figure 8, on propose des surfaces articulaires métacarpienne 204 et trapézienne 206, qui correspondent chacune à un segment axial d'un cône, centré sur un axe X₂₀₄, X₂₀₆ et dont la section transversale S₂₀₄, S₂₀₆ est circulaire. A titre de variante non représentée, les sections précitées S₂₀₄ et S₂₀₆ peuvent être elliptiques.

Egalement de même, sur la figure 9, on propose des surfaces articulaires métacarpienne 304 et trapézienne 306, qui correspondent chacune à un segment axial d'un tore, qui est associé à un axe central circulaire X₃₀₄, X₃₀₆ et dont la section transversale S₃₀₄, S₃₀₆, c'est-à-dire la section dans un plan perpendiculaire à l'axe précité, est circulaire centrée sur cet axe.

A titre de variantes non représentées, la section transversale S₃₀₄, S₃₀₆ du tore précité peut être prévue elliptique. De même, également à titre de variante non représentée, plutôt que d'être constante suivant l'axe X₃₀₄, X₃₀₆, la section circulaire ou la section elliptique de ce tore peut augmenter ou diminuer suivant l'axe.

En outre, également à titre de variantes, la géométrie incurvée des sections S₁₀₄, S₁₀₆, S₂₀₄, S₂₀₆, S₃₀₄ et S₃₀₆ peut soit être rigoureusement circulaire ou elliptique, soit, au contraire, présenter des variations de courbure suivant sa périphérie. En particulier, comme évoqué plus haut pour la géométrie elliptique des sections S₄ et S₆, la géométrie circulaire ou elliptique des sections S₁₀₄, S₁₀₆, S₂₀₄, S₂₀₆, S₃₀₄ et/ou S₃₀₆ peut présenter une courbure plus petite dans la région centrale des surfaces articulaires correspondantes 104, 106, 204, 206, 304, 306 et/ou présenter une courbure plus grande dans la région périphérique de cette surface articulaire, par rapport au reste de cette section transversale.

Les figures 6 à 9 illustrent ainsi la multiplicité des géométries de construction des surfaces articulaires métacarpienne et trapézienne de l'implant 1 : ces diverses géométries de construction fournissent toutes une mobilité articulaire proche de la mobilité anatomique de l'articulation trapézo-métacarpienne une fois que le corps 2 est implanté, du moment que les deux surfaces articulaires opposées 4 et 6, 104 et 106, 204 et 206, et 304 et 306 de l'implant 1 sont agencées orthogonalement l'une à l'autre, c'est-à-dire de telle sorte que leurs axes centraux X₄ et X₆, X₁₀₄ et X₁₀₆, X₂₀₄ et X₂₀₆, et X₃₀₄ et X₃₀₆ s'étendent de manière sensiblement perpendiculaire l'un à l'autre. On notera que, dans le cas des axes courbes X₃₀₄ et X₃₀₆, cette caractéristique de perpendicularité s'apprécie par projection de ces axes dans le plan médian π du corps 2 de l'implant 1, étant remarqué que, pour toutes les formes de réalisation envisagées ci-dessus, au moins les régions centrales respectives des surfaces 304 et 306 sont disposées de part et d'autre de ce plan π. Bien entendu, les projections sur le plan π des axes rectilignes X₄ et X₆, X₁₀₄ et X₁₀₆, et X₂₀₄ et X₂₀₆ sont, elles aussi, perpendiculaires l'une à l'autre.

On comprend également que la géométrie de construction relative à la surface articulaire métacarpienne, choisie parmi toutes celles évoquées ci-dessus, peut être la même, avec un dimensionnement rigoureusement identique ou différent, ou bien être différente de la géométrie de construction choisie pour la surface articulaire trapézienne.

## Revendications

1. Implant d'articulation trapézo-métacarpienne,
comportant un corps (2) définissant une surface articulaire métacarpienne (4 ; 104 ; 204 ; 304) et une surface articulaire trapézienne (6 ; 106 ; 206 ; 306), qui sont situées, au moins pour leur région centrale (42, 62), de part et d'autre d'un plan médian (π) du corps, **caractérisé en ce que** les surfaces articulaires métacarpienne (4 ; 104 ; 204 ; 304) et trapézienne (6 ; 106 ; 206 ; 306) correspondent chacune à un segment axial de cylindre, de cône ou de tore, ces deux segments axiaux de cylindre, de cône et/ou de tore étant centrés sur des axes respectifs (X₄, X₆ ; X₁₀₄, X₁₀₆; X₂₀₄, X₂₀₆ ; X₃₀₄, X₃₀₆) qui, projetés sur le plan médian, sont sensiblement perpendiculaires l'un à l'autre.

2. Implant suivant la revendication 1, **caractérisé en ce que** l'une ou chacune des surfaces articulaires métacarpienne (4 ; 104 ; 204 ; 304) et trapézienne (6 ; 106 ; 206 ; 306) correspond à un segment axial de cylindre ou de cône ou de tore, à section transversale (S₄, S₆ ; S₁₀₄, S₁₀₆ ; S₂₀₄, S₂₀₆ ; S₃₀₄, S₃₀₆) incurvée sur toute sa périphérie.

3. Implant suivant la revendication 2, **caractérisé en ce que** l'une ou chacune des surfaces articulaires métacarpienne (4 ; 204 ; 304) et trapézienne (6 ; 206 ; 306) correspond à un segment axial de cylindre ou de cône ou de tore, à section transversale elliptique.

4. Implant suivant l'une des revendications 2 ou 3, **caractérisé en ce que** l'une ou chacune des surfaces articulaires métacarpienne (104 ; 204 ; 304) et trapézienne (106 ; 206 ; 306) correspond à un segment axial de cylindre ou de cône ou de tore, à section transversale circulaire.

5. Implant suivant l'une quelconque des revendications 2 à 4, **caractérisé en ce que** la section transversale (S₄, S₆) associée à la surface articulaire métacarpienne (4) et/ou à la surface articulaire trapézienne (6) présente, dans la région centrale (42, 62) de cette surface articulaire, une courbure plus petite que le reste de la section transversale.

6. Implant suivant l'une quelconque des revendications 2 à 5, **caractérisé en ce que** la section transversale (S₄, S₆) associée à la surface articulaire métacarpienne (4) et/ou à la surface articulaire trapézienne (6) présente, dans la région périphérique (43, 63) de cette surface articulaire, une courbure plus grande que le reste de la section transversale.

7. Implant suivant l'une quelconque des revendications précédentes, **caractérisé en ce que** les surfaces articulaires métacarpienne (4 ; 104 ; 204 ; 304) et trapézienne (6 ; 106 ; 206 ; 306) sont séparées l'une de l'autre, suivant une direction (Z-Z) perpendiculaire au plan médian (π), par une épaisseur maximale (e₂) du corps (2) inférieure à 5 mm.

8. Implant suivant l'une quelconque des revendications précédentes, **caractérisé en ce que** la surface articulaire trapézienne (6 ; 106 ; 206 ; 306) présente une dimension (L₆), suivant son axe associé (X₆ ; X₁₀₆ ; X₂₀₆ ; X₃₀₆), supérieure à la dimension (L₄), suivant son axe associé (X₄ ; X₁₀₄ ; X₂₀₄ ; X₃₀₄), de la surface articulaire métacarpienne (4 ; 104 ; 204 ; 304).

9. Implant suivant l'une quelconque des revendications précédentes, **caractérisé en ce que** le corps (2) est dépourvu de moyen de fixation osseuse.

10. Implant suivant l'une quelconque des revendications précédentes, **caractérisé en ce que** l'implant (1) est constitué exclusivement du corps (2) réalisé d'un seul tenant.

## Claims

1. Trapezo-metacarpal joint implant,
comprising a body (2) defining a metacarpal articular surface (4; 104; 204; 304) and a trapezial articular surface (6; 106; 206; 306), which are situated, at least as regards their central region (42, 62), on either side of a median plane (π) of the body, **characterised in that** the metacarpal (4; 104; 204; 304) and trapezial (6; 106; 206; 306) articular surfaces each correspond to an axial segment of a cylinder, a cone or a torus, the said two axial segments of a cylinder, a cone and/or a torus being centred on respective axes (X₄, X₆; X₁₀₄, X₁₀₆; X₂₀₄, X₂₀₆; X₃₀₄, X₃₀₆) which, projected onto the median plane, are substantially perpendicular to one another.

2. Implant according to claim 1, **characterised in that** one or each of the metacarpal (4; 104; 204; 304) and trapezial (6; 106; 206; 306) articular surfaces correspond(s) to an axial segment of a cylinder, or a cone or a torus with a transverse section (S₄, S₆; S₁₀₄, S₁₀₆; S₂₀₄, S₂₀₆; S₃₀₄, S₃₀₆) which is curved over its entire periphery.

3. Implant according to claim 2, **characterised in that** one or each of the metacarpal (4; 204; 304) and trapezial (6; 206; 306) articular surfaces correspond(s) to an axial segment of a cylinder, or a cone or a torus with an elliptical transverse section.

4. Implant according to either of claims 2 or 3, **characterised in that** one or each of the metacarpal (104; 204; 304) and trapezial (106; 206; 306) articular surfaces correspond(s) to an axial segment of a cylinder, or a cone or a torus with a circular transverse section.

5. Implant according to any of claims 2 to 4, **characterised in that** the transverse section (S₄, S₆) associated with the metacarpal articular surface (4) and/or the trapezial articular surface (6) has, in the central region (42, 62) of the said articular surface, a curvature which is smaller than the rest of the transverse section.

6. Implant according to any of claims 2 to 5, **characterised in that** the transverse section (S₄, S₆) associated with the metacarpal articular surface (4) and/or with the trapezial articular surface (6) has, in the peripheral region (43, 63) of the said articular surface, a curvature which is greater than the rest of the transverse section.

7. Implant according to any of the preceding claims, **characterised in that** the metacarpal (4; 104; 204; 304) and trapezial (6; 106; 206; 306) articular surfaces are separated from one another, in a direction (Z-Z) perpendicular to the median plane (π), by a maximum thickness (e₂) of the body (2) which is less than 5 mm.

8. Implant according to any of the preceding claims, **characterised in that** the trapezial articular surface (6; 106; 206; 306) has a dimension (L₆), along its associated axis (X₆; X₁₀₆; X₂₀₆; X₃₀₆) , which is greater than the dimension (L₄), along its associated axis (X₄; X₁₀₄; X₂₀₄; X₃₀₄) , of the metacarpal articular surface (4; 104; 204; 304).

9. Implant according to any of the preceding claims, **characterised in that** the body (2) is devoid of a bone fixation means.

10. Implant according to any of the preceding claims, **characterised in that** the implant (1) is made up exclusively of the body (2) which is produced in one piece.

## Patentansprüche

1. Trapezo-metakarpales Gelenkimplantat, einen Körper (2) umfassend, der eine metakarpale Gelenkfläche (4; 104; 204; 204) und eine Trapez-Gelenkfläche (6; 106; 206; 306) definiert, die zumindest für ihren mittleren Bereich (42, 62) beidseitig einer Mittelebene zu des Körpers liegen, **dadurch gekennzeichnet, dass** die metakarpale Gelenkfläche (4; 104; 204; 304) und die Trapez-Gelenkfläche (6; 106; 206; 306) jede einem axialen Zylinder-, Konus- oder Torussegment entsprechen, wobei diese zwei axialen Zylinder-, Konus- und/oder Torussegmente auf je-Welllge Achsen (X₄, X₆; X₁₀₄, X₁₀₆; X₂₀₄, X₂₀₆; X₃₀₄, X₃₀₆) zentriert sind, die, projiziert auf die Mittelebene, im Wesentlichen senkrecht zueinander stehen.

2. Implantat nach Anspruch 1, **dadurch gekennzeichnet, dass** eine oder jede der beiden Flächen, der metakarpalen Gelenkfläche (4; 104; 204; 304) und der Trapez-Gelenkfläche (6; 106; 206; 306), einem axialen Zylinder- oder Konus- oder Torussegment mit einem über seinen gesamten Umfang gekrümmten Querschnitt (5₄, S₆; S₁₀₄, S₁₀₆; S₂₀₄, S₂₀₆; S₃₀₄, S₃₀₆) entsprechen.

3. Implantat nach Anspruch 2, **dadurch gekennzeichnet, dass** eine oder jede der beiden Flächen, der metakarpalen Gelenkfläche (4; 104; 204; 304) und der Trapez-Gelenkfiäche (6; 106; 206; 306), einem axialen Zylinder- oder Konus- oder Torussegment mit elliptischem Querschnitt entsprechen.

4. Implantat nach einem der Ansprüche 2 oder 3, **dadurch gekennzeichnet, dass** eine oder jede der beiden Flächen, der metakarpalen Gelenkfläche (4; 104; 204; 304) und der Trapez-Gelenkfläche (6; 106; 206; 306), einem axialen Zylinder- oder Konus- oder Torussegment mit kreisförmigem Querschnitt entsprechen.

5. Implantat nach einem beliebigen der Ansprüche 2 bis 4, **dadurch gekennzeichnet, dass** der Querschnitt (S₄, S₆), der der metakarpalen Gelenkfläche (4) und/oder der Trapez-Gelenkfläche (6) zugeordnet ist, im mittleren Bereich (42, 62) dieser Gelenkfläche eine Krümmung aufweist, die kleiner ist als der Rest des Querschnitts.

6. Implantat nach einem beliebigen der Ansprüche 2 bis 5, **dadurch gekennzeichnet, dass** der Querschnitt (S₄, S₆), der der metakarpalen Gelenkfläche (4) und/oder der Trapez-Gelenkfläche (6) zugeordnet ist, im Umfangsbereich (43, 63) dieser Gelenkfläche eine Krümmung aufweist, die größer ist als der Rest des Querschnitts.

7. Implantat nach einem beliebigen der vorhergehenden Ansprüche, **dadurch gekennzeichnet, dass** die metakarpale Gelenkfläche (4; 104; 204; 304) und die Trapez-Gelenkfläche (6; 106; 206; 306) gemäß einer Richtung (Z-Z) senkrecht zur Mittelebene (π) durch eine maximale Dicke (e₂) des Körpers (2) kleiner als 5 mm voneinander getrennt sind.

8. Implantat nach einem beliebigen der vorhergehenden Ansprüche, **dadurch gekennzeichnet, dass** die Trapez-Gelenkfläche (6; 106; 206; 306) eine Abmessung (L₆) gemäß ihrer zugeordneten Achse (X₆; X₁₀₆; X₂₀₆; X₃₀₆) aufweist, die größer ist als die Abmessung (L₄) gemäß ihrer zugeordneten Achse (X₄; X₁₀₄; X₂₀₄; X₃₀₄) der metakarpalen Gelenkfläche (4; 104, 204; 304).

9. Implantat nach einem beliebigen der vorhergehenden Ansprüche, **dadurch gekennzeichnet, dass** der Körper (2) keine Knochenbefestigungsmittel aufweist.

10. Implantat nach einem beliebigen der vorhergehenden Ansprüche, **dadurch gekennzeichnet, dass** das Implantat (1) nur aus dem Körper (2) besteht, der aus einem einzigen Stück hergestellt ist.
